# FASCICULE DE BREVET EUROPEEN

(11) **EP 0 680 747 B1**
(45) Date de publication et mention de la délivrance du brevet: **01.07.1998**
(21) Numéro de dépôt: 95400598.9
(22) Date de dépôt: 17.03.1995
(51) Int. Cl.: A61K 7/48

(54) **Composition cosmétique et/ou dermatologique contenant un tri(alpha-hydroxyacylate) de glycérol comme seul précurseur de glycérol**
Kosmetische und/oder dermatologische Zusammensetzung, die ein Tri (alpha-hydroxyacylat) von glycerin als einzigen Glycerinprecursor enthält
Cosmetic and/or dermatologic composition containing a glycerol tri(alpha-hydroxyacylate) as sole precursor of glycerol

(30) Priorité: 18.04.1994 FR 9404602
(43) Date de publication de la demande: 08.11.1995
(73) Titulaire: L'OREAL, 75008 Paris (FR)
(72) Inventeur: Laugier, Jean-Pierre, F-92160 Antony (FR); Nadaud, Jean-François, F-92140 Clamart (FR)
(74) Mandataire: Lhoste, Catherine

(56) Documents cités:
- GB-A- 2 092 444
- US-A- 4 675 177
- US-A- 4 760 096

## Description

La présente invention a pour objet l'utilisation d'un tri(α-hydroxyacylate) de glycérol dans une composition cosmétique et/ou dermatologique pour hydrater et/ou assouplir la peau, aussi bien du visage que du corps, voire même du cuir chevelu.

Elle a aussi pour objet un procédé de traitement cosmétique par voie topique, pour hydrater et/ou assouplir la peau.

On sait que la peau a tendance à se dessécher du fait de son exposition à l'air et au soleil ; la perte en eau en surface entraîne également une perte en eau dans le *stratum corneum*. De ce fait, il est important que la peau soit bien hydratée et ne subisse pas de perte en eau risquant d'entraîner un flétrissement de la peau, et donc son vieillissement prématuré, un dessèchement, voire une desquamation. Aussi, dans le domaine cosmétique, il est courant d'incorporer dans les compositions utilisées des hydratants, c'est-à-dire des substances hygroscopiques qui provoquent une réhydratation de la peau par captation de l'eau atmosphérique et par rétention de l'eau de la peau.

Parmi les nombreux hydratants, on utilise le plus couramment les polyols, et notamment le glycérol qui est un capteur d'eau du fait de ses groupements hydroxyle ; ainsi une molécule de glycérol capte six molécules d'eau. De plus, sa molécule est peu encombrante, ce qui lui permet de se faufiler dans la peau. On peut citer à ce sujet le paragraphe sur la glycérine dans The Principles and Practice of Modern Cosmetics de R. G. Harry, 1963, volume II pages 202 à 205.

Le seul inconvénient du glycérol est d'apporter un caractère collant aux compositions qui le contiennent en grande quantité.

Par ailleurs, il est connu aussi d'utiliser les acides hydroxylés et leurs sels, et notamment l'acide lactique et le lactate de sodium, ce dernier étant un des composants du NMF (Natural Moisturizing Factor) présent dans la peau ; on pense, en effet, que l'acide lactique ou son sel modifie la conformation spatiale des protéines du *stratum corneum*. De ce fait, il améliore la souplesse et l'élasticité de la peau. On peut citer à ce sujet l'article de M. Rieger, Cosmetics & Toiletries, 1992, vol. 107, pp.89-90.

Néanmoins, les acides hydroxylés et leurs sels présentent l'inconvénient d'être difficiles à incorporer dans une composition cosmétique et/ou dermatologique, surtout quand on veut les utiliser en quantité élevée, car ils sont alors incompatibles avec la plupart des gélifiants habituellement utilisés pour stabiliser les compositions. Cette incompatibilité se traduit par une déstabilisation des compositions qui les contiennent. Dans le cas d'une émulsion, il se produit alors une séparation des phases aqueuse et huileuse, et dans le cas d'un gel, il casse.

Pour résoudre ce problème, on peut soit les incorporer en de très faibles quantités, soit les utiliser dans des supports bien particuliers qui sont compatibles, ce qui, dans les deux cas, limite la liberté de formulation. Ainsi, l'acide lactique ou le lactate de sodium sont toujours utilisés avec les mêmes types de gélifiant et d'émulsionnant, et on obtient donc toujours des compositions de même texture, le plus souvent grasses et cireuses. Il est donc très difficile d'utiliser les acides hydroxylés et leurs sels dans n'importe quel type de composition. En particulier, il n'est pas possible d'obtenir des laits et des lotions.

L'invention a justement pour objet une composition cosmétique et/ou dermatologique permettant de résoudre les problèmes mentionnés ci-dessus.

La demanderesse a découvert de manière surprenante que l'utilisation d'un tri(α-hydroxyacylate) de glycérol dans une composition cosmétique et/ou dermatologique permettait d'obtenir au moins les mêmes effets sur la peau que le glycérol et l'acide hydroxylé correspondant tout en permettant l'utilisation de tout type de gélifiant et d'émulsionnant, et donc l'obtention de nouvelles formes galéniques jusque-là impossibles à stabiliser en présence d'acide hydroxylé, telles que les gels aqueux et les émulsions fluides.

Certes, le document US-A-4675177 décrit des compositions antitranspirantes contenant des esters de polyol pour faciliter l'activation de l'astringent. Toutefois, ce document ne suggère pas que les triesters de polyol puissent être utilisés pour hydrater et assouplir la peau.

Les tri(α-hydroxyacylates) de glycérol présentent en outre l'avantage d'être faciles à introduire dans les compositions cosmétiques et/ou dermatologiques, et de donner des compositions moins collantes que celles qui contiennent une quantité équivalente de glycérol. En particulier, les tri(α-hydroxyacylates) de glycérol sont moins hydrophiles que les acides hydroxylés et réagissent moins avec les constituants de la composition les contenant.

L'invention a donc pour objet l'utilisation d'un tri(α-hydroxyacylate) de glycérol dans une composition cosmétique et/ou dermatologique comme seul précurseur du glycérol et d'un acide hydroxylé, apte à libérer par réaction enzymatique sur la peau le glycérol et l'acide hydroxylé pour hydrater et/ou assouplir la peau.

On appelle précurseur d'actif un composé qui, au contact du *stratum corneum,* est hydrolysé par l'action d'un enzyme spécifique et libère l'actif libre. Un précurseur a généralement l'inconvénient de présenter une efficacité plus faible que celle de l'actif libre. Or, dans le cas du trilactate de glycérol, de manière surprenante, l'efficacité hydratante est identique à celle des actifs libérés, voire supérieure.

De manière caractéristique, il n'est utilisé selon la présente invention qu'un seul précurseur. On peut toutefois y associer d'autres actifs qui ne se trouvent pas sous forme de précurseurs bioconvertibles sur la peau.

En particulier, le tri(α-hydroxyacylate) de glycérol est un composé de formule : 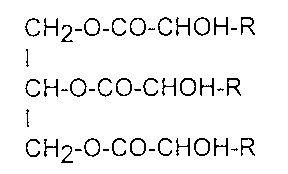 dans laquelle R représente le radical CₙH₂ₙ₊₁, n étant un nombre entier de 0 à 12.

En particulier, le tri(α-hydroxyacylate) de glycérol est le trilactate de glycérol, ou encore le triglycolate de glycérol, le trimandélate de glycérol, le tritartrate de glycérol ou le tricitrate de glycérol.

La composition selon l'invention contenant un tri(α-hydroxyacylate) de glycérol permet de lutter contre la deshydratation et le dessèchement de la peau, et par conséquent contre son vieillissement.

L'invention a donc aussi pour objet un procédé de traitement cosmétique pour hydrater et/ou assouplir la peau, consistant à appliquer sur la peau une composition contenant un tri(α-hydroxyacylate) de glycérol.

L'invention a encore pour objet l'utilisation d'un tri(α-hydroxyacylate) de glycérol pour la préparation d'une composition dermatologique pour traiter la peau sèche. La composition selon l'invention peut contenir, par exemple, de 0,01 à 20 % en poids de tri(α-hydroxyacylate) de glycérol par rapport au poids total de la composition, et de préférence de 0,5 à 10 % en poids.

La composition selon l'invention peut se présenter sous toutes les formes galéniques normalement utilisées pour une application topique, et par exemple sous forme d'une lotion aqueuse ou hydroalcoolique, sous forme d'un gel aqueux, sous forme d'un sérum, sous forme d'une émulsion huile-dans-eau (H/E) ou eau-dans-huile (E/H). Elle peut se présenter aussi sous forme de sphérules comme les vésicules de lipides ioniques (liposomes) ou non-ioniques, les nanocapsules, les nanosphères.

En particulier, les émulsions peuvent se présenter sous forme de laits qui sont des émulsions fluides. Une émulsion fluide présente en pratique une viscosité inférieure à 8 poises (0,8 Pa.s), mesurée au viscosimètre Contraves MTV, c'est-à-dire qui s'écoule sous son propre poids.

Il est également particulièrement intéressant de pouvoir obtenir des gels aqueux contenant peu ou pas d'huile. On peut utiliser pour ce faire tout type de gélifiant, et par exemple un polymère carboxyvinylique (carbomer), un copolymère acrylique tel que les copolymères d'acrylates / alkylacrylates, un polyacrylamide ou un polysaccharide.

Lorsque la composition est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont alors présents, dans la composition, en une proportion allant de 0,3 % à 30 % en poids, et de préférence de 0,5 à 30 % en poids par rapport au poids total de la composition. L'émulsion peut, en outre, contenir des vésicules lipidiques.

De façon connue, la composition cosmétique et/ou dermatologique de l'invention peut contenir également des adjuvants habituels dans le domaine cosmétique et/ou dermatologique, tels que les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans le domaine cosmétique et/ou dermatologique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (huile de tournesol), les huiles de synthèse (myristate d'isopropyle, perhydrosqualène, lanoline, huile de Purcellin), les huiles siliconées (cyclométhicone) et les huiles fluorées (perfluoropolyéthers). On peut ajouter à ces huiles des alcools gras et des acides gras (alcool cétylique, acide stéarique, alcool de lanoline).

Comme émulsionnant E/H utilisable dans l'invention, on peut citer par exemple le mélange de polyglycéryl-4 isostéarate/cétyldiméthicone copolyol/hexyl laurate vendu sous la dénomination ABIL WE 09 par la société Goldschmidt et le mélange Mineral oil/petrolatum/ozokérite/glycéryl oléate/alcool de lanoline vendu sous la dénomination PROTEGIN X par la société Goldschmidt.

Comme émulsionnant H/E utilisable dans l'invention, on peut citer par exemple le mélange de glycéryl stéarate/PEG-100 stéarate vendu sous la dénomination ARLACEL 165 par la société ICI, le polysorbate-60 vendu sous la dénomination TWEEN 60 par la société ICI, le PEG-400, le stéarate de glycéryle, le mélange de PEG-6/PEG-32/glycol stéarate vendu sous la dénomination TEFOSE 63 par la société Gattefosse, le mélange de PEG-100 stéarate/Glycéryl stéarate vendu sous la dénomination SIMULSOL 165 par la société Seppic.

Comme gélifiants hydrophiles, on peut citer ceux indiqués ci-dessus ainsi que les gommes naturelles et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser les protéines ou les hydrolysats de protéine, les acides aminés, les polyols, l'urée, l'allantoïne, les sucres et les dérivés de sucre, les vitamines et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

On peut aussi utiliser dans la composition selon l'invention des filtres UV à propriété lipophile ou hydrophile, et les oxydes de titane et de zinc.

Les tests suivants montrent les résultats obtenus en hydratation par le trilactate de glycérol comparativement au glycérol et au lactate de sodium.

L'hydratation a été mesurée d'une part au cornéomètre qui mesure la capacitance de la peau in vivo, d'autre part au dermodiag qui mesure la conductance de la peau in vivo. Les deux mesures se complètent pour rendre compte de l'hydratation d'une substance.

Les résultats sont rassemblés dans le tableau suivant, où les pourcentages indiqués représentent l'augmentation de capacitance ou de conductance par rapport à celles trouvées pour la peau nue :

| | Excipient | Glycérol à 5 % | Trilactate de glycérol à 5% | Lactate de sodium à 3% |
|---|---|---|---|---|
| Cornéomètre | + 9 % | + 22 % | + 22 % | + 23 % |
| Dermodiag | + 10 % | + 25 % | + 20 % | + 5 % |

5 % de trilactate de glycérol correspondent à 1,25 % de glycérol et 3,75 % de lactate, et 3 % de lactate de sodium correspondent à environ 2,7 % de lactate.

Les résultats présentés dans le tableau ci-dessus montrent que le trilactate de glycérol fournit globalement une hydratation équivalente à celle d'une quantité quatre fois plus grande de glycérol et est plus importante qu'une quantité correspondante de lactate.

### Exemple 1 : Emulsion de type H/E

| *Phase A :* | |
|---|---|
| - Cyclomethicone | 10 g |
| - Perhydrosqualène | 18 g |
| - Huile de Vaseline | 5 g |
| - Lanoline Liquide | 4 g |
| - Glycéryl stéarate/PEG-100 stéarate (Arlacel^{R} 165 de Ici) | 6 g |
| - Polysorbate 60 (Tween^{R} 60 de Ici) | 2 g |
| - Alcool Cétylique | 1,2 g |
| - Acide Stéarique | 2,5 g |

| *Phase B :* | |
|---|---|
| - Triéthanolamine | 0,1 g |
| - Conservateur | 0,3 g |
| - Antioxydants | 0,3 g |
| - Trilactate de Glycérol | 5 g |
| - Eau déminéralisée | qsp 100 g |

### Mode opératoire :

On chauffe la *phase A* à 75 °C. On chauffe de même la *phase B* et on verse la *phase A* dans la *phase B* sous agitation. On refroidit jusqu'à la température ambiante. On obtient une crème de soin hydratante, onctueuse, à utiliser plutôt le jour.

### Exemple 2 : Emulsion de type H/E

| *Phase A :* | |
|---|---|
| - Parfum | 0,5 g |
| - Carbomer | 0,2 g |
| - Myristate d'Isopropyle | 1 g |
| - Alcool Cétylique | 3 g |
| - Acide Stéarique | 3 g |
| - Stéarate de Glycéryle | 3 g |
| - Huile de Germe de maïs | 2 g |

| *Phase B :* | |
|---|---|
| - Trilactate de Glycérol | 5 g |
| - Propylène Glycol | 2 g |
| - PEG 400 | 3 g |
| - Conservateur | 0,3 g |
| - Eau déminéralisée | qsp 100 g |

On procède comme dans l'exemple 1. On obtient une crème de soin hydratante comme dans l'exemple 1.

### Exemple 3 : Emulsion de type E/H

| *Phase A :* | |
|---|---|
| - Mineral oil/Petrolatum/ozokerite/Glycéryl oléate/Lanoline alcool (Protegin X de Goldschmidt) | 20 g |
| - Huile de Vaseline | 10 g |
| - Parfum | 1 g |
| - Huile de Tournesol | 15 g |

| *Phase B :* | |
|---|---|
| - Conservateur | 0,3 g |
| - Glycérol | 5 g |
| - Sulfate de Magnésium | 0,5 g |
| - Trilactate de Glycérol | 5 g |
| - Eau déminéralisée | qsp 100 g |

### Mode opératoire :

On chauffe séparément les *phases A* et *B* à 75 °C. On verse ensuite la *phase B* dans la *phase A*. On obtient une crème nourrissante ayant de bonnes propriétés hydratantes, utilisable plutôt la nuit.

### Exemple 4 : Emulsion de type E/H

| *Phase A :* | |
|---|---|
| - Polyglycéryl - 4 isostéarate/Cétyldimethicone-copolyol/hexyl laurate (Abil WE 09 de Goldschmidt) | 5 g |
| - Myristate d'Isopropyle | 5 g |
| - Cyclomethicone | 8 g |
| - Huile de Vaseline | 5 g |
| - Silice (Aérosil^{R} 200 de Degussa) | 0,4 g |
| - Huile de Purcellin (vendue par la Société Stéarineries Dubois) | 14 g |

| *Phase B :* | |
|---|---|
| - Chlorure de Sodium | 0,5 g |
| - Conservateur | 0,3 g |
| - Trilactate de Glycérol | 5 g |
| - Eau déminéralisée | qsp 100 g |

On procède comme dans l'exemple précédent et on obtient une crème de nuit hydratante.

### Exemple 5 : Gel Hydroalcoolique

| | |
|---|---|
| - Carbomer | 0,9 g |
| - Alcool Ethylique | 10 g |
| - Triéthanolamine | 0,3 g |
| - Parfum | 0,3 g |
| - Conservateur | 0,3 g |
| - Trilactate de Glycérol | 5 g |
| - Eau déminéralisée | qsp 100 g |

On obtient un tonique hydratant à utiliser pour nettoyer la peau.

### Exemple 6 : Gel Emulsionné H/E

| *Phase A :* | |
|---|---|
| - Cyclomethicone | 3 g |
| - Huile de Purcellin (vendue par la Société Dragocco) | 7 g |
| - PEG-6/PEG-32/Glycol Stéarate (Tefose^{R} 63 de Gattefosse) | 0,3 g |
| - Conservateur | 0,3 g |
| - Parfum | 0,4 g |

| *Phase B :* | |
|---|---|
| Carbomer | 0,6 g |
| - Alcool Ethylique | 10 g |
| - Triéthanolamine | 0,2 g |
| - Trilactate de Glycérol | 5 g |
| - Eau déminéralisée | qsp 100 g |

Pour la préparation, on procède comme dans l'exemple 1, en chauffant les *phases A* et *B* respectivement à 60 °C et 40 °C. On obtient un gel utilisable pour hydrater et nettoyer la peau.

### Exemple 7 : Gel

| | |
|---|---|
| - Carbomer | 0,6 g |
| - Triéthanolamine | 0,3 g |
| - Conservateur | 0,3 g |
| - Propylène Glycol | 3 g |
| - Trilactate de Glycérol | 5 g |
| - Eau déminéralisée | qsp 100 g |

On obtient un gel hydratant adapté aux peaux sensibles.

### Exemple 8 : Crème aux Vésicules non ioniques

| *Phase A :* | |
|---|---|
| - Polyglyceryl-3 Cétyl éther | 3,8 g |
| - β - sitostérol | 3,8 g |
| - Dicétyl-Phosphate | 0,4 g |

| *Phase B :* | |
|---|---|
| - Conservateur | 0,3 g |
| - Huile de Tournesol | 35 g |
| - Parfum | 0,6 g |

| *Phase C :* | |
|---|---|
| - Carbomer | 0,2 g |
| - Triéthanolamine | 0,2 g |
| - Trilactate de Glycérol | 5 g |
| - Eau déminéralisée | qsp 100 g |

### Mode opératoire :

On fait fondre les constituants de la *phase A* à 100 °C, on y ajoute de l'eau sous agitation et on fait deux passages à l'homogénéisateur, à haute pression, pour former les vésicules.

On chauffe à 70 °C les constituants de la *phase B* jusqu'à solubilisation complète. On refroidit et on ajoute la *phase B* à la *phase A*.

Après deux passages à l'homogénéisateur à haute pression, on refroidit et on ajoute la phase C sous agitation.

On obtient une crème nourrissante et hydratante.

### Exemple 9 : Emulsion de type H/E

| *Phase A :* | |
|---|---|
| - Cyclomethicone | 5 g |
| - Alcool de Lanoline | 1,5 g |
| - Huile de Vaseline | 10 g |
| - PEG-100 Stéarate/Glycéryl stéarate (Simulsol^{R} 165 de Seppic) | 2,1 g |
| - Polysorbate-60 (Tween^{R} 60 de Ici) | 0,9 g |
| - Alcool Cétylique | 0,5 g |
| - Acide Stéarique | 0,72 g |

| *Phase B :* | |
|---|---|
| Carbomer | 0,2 g |
| - Gomme de Xanthane | 0,1 g |
| - Triéthanolamine | 0,45 g |
| - Conservateur | 1,16 g |
| - Trilactate de Glycérol | 5 g |
| - Eau déminéralisée | qsp 100 g |

On procède comme dans l'exemple 1 et on obtient une crème comparable à celle de l'exemple 1.

### Exemple 10 : Emulsion de type H/E

| *Phase A :* | |
|---|---|
| - Cyclomethicone | 10 g |
| - Alcool de Lanoline | 1,5 g |
| - Huile de Vaseline | 5 g |
| - PEG-100 Stéarate/Glycéryl stéarate (Simulsol^{R} 165 de Seppic) | 2,1 g |
| - Polysorbate-60 (Tween^{R} 60 de Ici) | 0,9 g |
| - Alcool Cétylique | 0,5 g |
| - Acide Stéarique | 0,72 g |

| *Phase B :* | |
|---|---|
| Carbomer | 0,2 g |
| - Gomme de Xanthane | 0,1 g |
| - Triéthanolamine | 0,45 g |
| - Glycérine | 3 g |
| - Conservateur | 1,1 g |
| - Trilactate de Glycérol | 2 g |
| - Eau déminéralisée | qsp 100 g |

La préparation et la crème obtenues sont identiques à celles de l'exemple 1.

## Revendications

1. Utilisation d'un tri(α-hydroxyacylate) de glycérol dans ou pour la fabrication d'une composition cosmétique et/ou dermatologique comme seul précurseur du glycérol et d'un acide hydroxylé, apte à libérer par réaction enzymatique sur la peau le glycérol et l'acide hydroxylé, pour hydrater et/ou assouplir la peau.

2. Utilisation selon la revendication 1, caractérisée en ce que le tri(α-hydroxyacylate) de glycérol est un composé de formule : 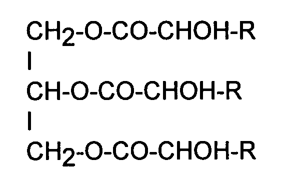 dans laquelle R représente le radical CₙN₂ₙ₊₁, n étant un nombre entier de 0 à 12.

3. Utilisation selon la revendication 1 ou 2, caractérisée en ce que le tri(α-hydroxyacylate) de glycérol est choisi parmi le trilactate de glycérol, le triglycolate de glycérol, le trimandélate de glycérol, le tritartrate de glycérol et le tricitrate de glycérol.

4. Utilisation selon l'une quelconque des revendications précédentes, caractérisée en ce que le tri(α-hydroxyacylate) de glycérol est le trilactate de glycérol.

5. Utilisation selon l'une quelconque des revendications précédentes, caractérisée en ce que la composition contient de 0,01 à 20 % en poids de tri(α-hydroxyacylate) de glycérol par rapport au poids total de la composition.

6. Utilisation selon l'une quelconque des revendications précédentes, caractérisée en ce que la composition contient de 0,5 à 10 % en poids de tri(α-hydroxyacylate) de glycérol par rapport au poids total de la composition.

7. Utilisation selon l'une quelconque des revendications précédentes, caractérisée en ce que la composition se présente sous forme de gel aqueux.

8. Utilisation selon l'une quelconque des revendications précédentes, caractérisée en ce que la composition se présente sous forme d'une émulsion fluide.

9. Procédé de traitement cosmétique pour hydrater et assouplir la peau consistant à appliquer sur la peau une composition contenant un tri(α-hydroxyacylate) de glycérol.

10. Procédé selon la revendication 9, caractérisé en ce que le tri(α-hydroxyacylate) de glycérol est un composé de formule : 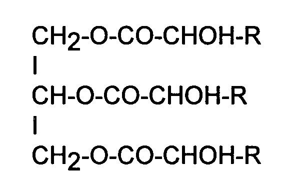 dans laquelle R représente le radical CₙH₂ₙ₊₁, n étant un nombre entier de 0 à 12.

11. Procédé selon la revendication 9 ou 10, caractérisé en ce que le tri(α-hydroxyacylate) de glycérol est choisi parmi le trilactate de glycérol, le triglycolate de glycérol, le trimandélate de glycérol, le tritartrate de glycérol et le tricitrate de glycérol.

12. Procédé de traitement cosmétique pour hydrater et assouplir la peau selon la revendication 11 consistant à appliquer sur la peau une composition contenant du trilactate de glycérol.

13. Utilisation d'un tri(α-hydroxyacylate) de glycérol pour la préparation d'une composition dermatologique pour traiter la peau sèche.

14. Utilisation selon la revendication 13, caractérisée en ce que le tri(α-hydroxyacylate) de glycérol est un composé de formule : 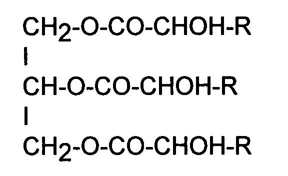 dans laquelle R représente le radical CₙH₂ₙ₊₁, n étant un nombre entier de 0 à 12.

15. Utilisation selon la revendication 13 ou 14, caractérisée en ce que le tri(α-hydroxyacylate) de glycérol est choisi parmi le trilactate de glycérol, le triglycolate de glycérol, le trimandélate de glycérol, le tritartrate de glycérol et le tricitrate de glycérol.

16. Utilisation selon l'une quelconque des revendications 13 à 15, caractérisée en ce que la composition contient de 0,01 à 20 % en poids de tri(α-hydroxyacylate) de glycérol par rapport au poids total de la composition.

17. Utilisation selon l'une quelconque des revendications 13 à 16, caractérisée en ce que la composition contient de 0,5 à 10 % en poids de tri(α-hydroxyacylate) de glycérol par rapport au poids total de la composition.

## Claims

1. Use of a glyceryl tri(α-hydroxyacylate) in, or for the manufacture of, a cosmetic and/or dermatological composition as sole precursor of glycerol and of a hydroxy acid, which is capable of releasing the glycerol and the hydroxy acid onto the skin via an enzymatic reaction, in order to moisturize and/or soften the skin.

2. Use according to Claim 1, characterized in that the glyceryl tri(α-hydroxyacylate) is a compound of formula: 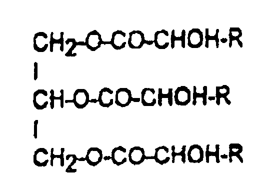 in which R represents the radical CₙH₂ₙ₊₁, n being an integer from 0 to 12.

3. Use according to Claim 1 or 2, characterized in that the glyceryl tri(α-hydroxyacylate) is chosen from glyceryl trilactate, glyceryl triglycolate, glyceryl trimandelate, glyceryl tritartrate and glyceryl tricitrate.

4. Use according to any one of the preceding claims, characterized in that the glyceryl tri(α-hydroxyacylate) is glyceryl trilactate.

5. Use according to any one of the preceding claims, characterized in that the composition contains from 0.01 to 20% by weight of glyceryl tri(α-hydroxyacylate) relative to the total weight of the composition.

6. Use according to any one of the preceding claims, characterized in that the composition contains from 0.5 to 10% by weight of glyceryl tri(α-hydroxyacylate) relative to the total weight of the composition.

7. Use according to any one of the preceding claims, characterized in that the composition is in the form of an aqueous gel.

8. Use according to any one of the preceding claims, characterized in that the composition is in the form of a fluid emulsion.

9. Cosmetic treatment process for moisturizing and softening the skin, consisting in applying to the skin a composition containing a glyceryl tri(α-hydroxyacylate).

10. Process according to Claim 9, characterized in that the glyceryl tri(α-hydroxyacylate) is a compound of formula: 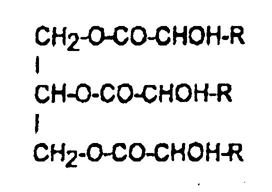 in which R represents the radical CₙH₂ₙ₊₁, n being an integer from 0 to 12.

11. Process according to Claim 9 or 10, characterized in that the glyceryl tri(α-hydroxyacylate) is chosen from glyceryl trilactate, glyceryl triglycolate, glyceryl trimandelate, glyceryl tritartrate and glyceryl tricitrate.

12. Cosmetic treatment process for moisturizing and softening the skin according to Claim 11, consisting in applying to the skin a composition containing glyceryl trilactate.

13. Use of a glyceryl tri(α-hydroxyacylate) for the preparation of a dermatological composition for treating dry skin.

14. Use according to Claim 13, characterized in that the glyceryl tri(α-hydroxyacylate) is a compound of formula: 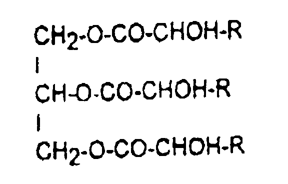 in which R represents the radical CₙH₂ₙ₊₁, n being an integer from 0 to 12.

15. Use according to Claim 13 or 14, characterized in that the glyceryl tri(α-hydroxyacylate) is chosen from glyceryl trilactate, glyceryl triglycolate, glyceryl trimandelate, glyceryl tritartrate and glyceryl tricitrate.

16. Use according to any one of Claims 13 to 15, characterized in that the composition contains from 0.01 to 20% by weight of glyceryl tri(α-hydroxyacylate) relative to the total weight of the composition.

17. Use according to any one of Claims 13 to 16, characterized in that the composition contains from 0.5 to 10% by weight of glyceryl tri(α-hydroxyacylate) relative to the total weight of the composition.

## Patentansprüche

1. Verwendung eines Tri(α-Hydroxyacylats) von Glycerin als einzigen Precursor von Glycerin und einer Hydroxysäure in einer kosmetischen und/oder dermatologischen Zusammensetzung oder zu deren Herstellung, um die Haut zu hydratisieren und/oder geschmeidig zu machen, wobei der Precursor befähigt ist, durch enzymatische Reaktion auf der Haut Glycerin und die Hydroxysäure freizusetzen.

2. Verwendung nach Anspruch 1, dadurch gekennzeichnet, daß das Tri(α-Hydroxyacylat) von Glycerin eine Verbindung der Formel 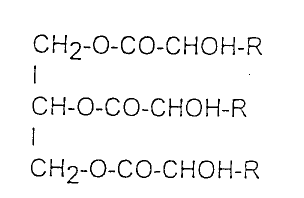 ist, worin R die Gruppe CₙH₂ₙ₊₁ bedeutet, wobei n eine ganze Zahl im Bereich von Null bis 12 ist.

3. Verwendung nach Anspruch 1 oder 2, dadurch gekennzeichnet, daß das Tri(α-Hydroxyacylat) von Glycerin unter Glycerintrilactat, Glycerintriglykolat, Glycerintrimandelat, Glycerintritartrat und Glycerintricitrat ausgewählt ist.

4. Verwendung nach einem der vorhergehenden Ansprüche, dadurch gekennzeichnet, daß das Tri(α-Hydroxyacylat) von Glycerin das Glycerintrilactat ist.

5. Verwendung nach einem der vorhergehenden Ansprüche, dadurch gekennzeichnet, daß die Zusammensetzung 0,01 bis 20 Gew.-% Tri(α-Hydroxyacylat) von Glycerin, bezogen auf das Gesamtgewicht der Zusammensetzung, enthält.

6. Verwendung nach einem der vorhergehenden Ansprüche, dadurch gekennzeichnet, daß die Zusammensetzung 5 bis 10 Gew.-% Tri(α-Hydroxyacylat) von Glycerin, bezogen auf das Gesamtgewicht der Zusammensetzung, enthält.

7. Verwendung nach einem der vorhergehenden Ansprüche, dadurch gekennzeichnet, daß die Zusammensetzung in Form eines wäßrigen Gels vorliegt.

8. Verwendung nach einem der vorhergehenden Ansprüche, dadurch gekennzeichnet, daß die Zusammensetzung in Form einer fluiden Emulsion vorliegt.

9. Verfahren zur kosmetischen Behandlung, um die Haut zu hydratisieren und geschmeidig zu machen, das darin besteht, auf die Haut eine Zusammensetzung aufzutragen, die ein Tri(α-Hydroxyacylat) von Glycerin enthält.

10. Verfahren nach Anspruch 9, dadurch gekennzeichnet, daß das Tri(α-Hydroxyacylat) von Glycerin eine Verbindung der Formel 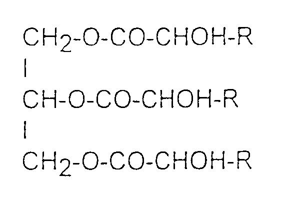 ist, worin R die Gruppe CₙH₂ₙ₊₁ bedeutet, wobei n eine ganze Zahl im Bereich von Null bis 12 ist.

11. Verfahren nach Anspruch 9 oder 10, dadurch gekennzeichnet, daß das Tri(α-Hydroxyacylat) von Glycerin unter Glycerintrilactat, Glycerintriglykolat, Glycerintrimandelat, Glycerintritartrat und Glycerintricitrat ausgewählt ist.

12. Verfahren zur kosmetischen Behandlung nach Anspruch 11, um die Haut zu hydratisieren und geschmeidig zu machen, das darin besteht, auf die Haut eine Zusammensetzung aufzutragen, die Glycerintrilactat enthält.

13. Verwendung eines Tri(α-Hydroxyacylats) von Glycerin zur Herstellung einer dermatologischen Zusammensetzung zur Behandlung von trockener Haut.

14. Verwendung nach Anspruch 13, dadurch gekennzeichnet, daß das Tri(α-Hydroxyacylat) von Glycerin eine Verbindung der Formel 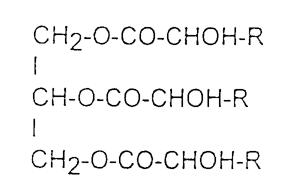 ist, worin R die Gruppe CₙH₂ₙ₊₁ bedeutet, wobei n eine ganze Zahl im Bereich von Null bis 12 ist.

15. Verwendung nach Anspruch 13 oder 14, dadurch gekennzeichnet, daß die Tri(α-Hydroxyacylat) von Glycerin unter Glycerintrilactat, Glycerintriglykolat, Glycerintrimandelat, Glycerintritartrat und Glycerincitrat ausgewählt ist.

16. Verwendung nach einem der Ansprüche 13 bis 15, dadurch gekennzeichnet, daß die Zusammensetzung 0,01 bis 20 Gew.-% Tri(α-Hydroxyacylat) von Glycerin, bezogen auf das Gesamtgewicht der Zusammensetzung, enthält.

17. Verwendung nach einem der Ansprüche 13 bis 16, dadurch gekennzeichnet, daß die Zusammensetzung 0,5 bis 10 Gew.-% Tri(α-Hydroxyacylat) von Glycerin, bezogen auf das Gesamtgewicht der Zusammensetzung, enthält.
